# EUROPEAN PATENT SPECIFICATION

(11) **EP 3 160 255 B1**
(45) Date of publication and mention of the grant of the patent: **27.11.2019**
(21) Application number: 14741519.4
(22) Date of filing: 26.06.2014
(51) Int. Cl.: A23K 50/10, A23K 50/60, A23K 20/163, A23K 20/142

(54) **BETAINE COMPOSITION, GLUCOSE COMPOSITION, AND USES THEREOF**
BETAINZUSAMMENSETZUNG, GLUCOSEZUSAMMENSETZUNG UND VERWENDUNGEN DAVON
COMPOSITION DE BÉTAÏNE, COMPOSITION DE GLUCOSE, ET LEURS UTILISATIONS

(43) Date of publication of application: 03.05.2017
(73) Proprietor: Tiense Suikerraffinaderij N.V., 1150 Brussel (BE); Südzucker AG Mannheim/Ochsenfurt, 68165 Mannheim (DE)
(72) Inventor: WACH, Wolfgang, 67549 Worms (DE); VAN LOO, Jan, B-3040 Huldenberg (BE)
(74) Representative: Koster, Nico
(86) International application number: PCT/EP2014/001748
(87) International publication number: WO 2015/197089

(56) References cited:
- WO-A1-2011/141175
- WO-A1-2013/072048
- BR-A2- PI0 705 359
- FR-A1- 2 851 425
- METZLER-ZEBELI BARBARA U ET AL: "Effects of betaine, organic acids and inulin as single feed additives or in combination on bacterial populations in the gastrointestinal tract of weaned", ARCHIVES OF ANIMAL NUTRITION, TAYLOR AND FRANCIS GROUP, US, vol. 63, no. 6, 1 December 2009 (2009-12-01), pages 427-441, XP008164841, ISSN: 1745-039X, DOI: 10.1080/17450390903299190 [retrieved on 2009-10-27]
- IRAJ GHAZI ET AL: "Beet Sugar Syrup and Molasses as Low-Cost Feedstock for the Enzymatic Production of Fructo-oligosaccharides", JOURNAL OF AGRICULTURAL AND FOOD CHEMISTRY, vol. 54, no. 8, 1 April 2006 (2006-04-01), pages 2964-2968, XP055134123, ISSN: 0021-8561, DOI: 10.1021/jf053023b
- BERTRAM, H.C. ET AL.: "NMR-based metabonomic studies reveal changes in the biochemical profile of plasma and urine from pigs fed high-fibre rye bread", BRITISH JOURNAL OF NUTRITION, vol. 95, 2006, pages 955-962, XP002728413,

## Description

The invention relates to a betaine composition and to a glucose composition. The invention further relates to methods for preparing betaine compositions and glucose compositions, and to uses of said compositions.

Betaine is as such known, and widely used in animal nutrition and other applications. Known betaine compositions are typically supplied in a rather high pure form, having about 90 wt.% betaine on total dry matter.

It is one objective of the present invention to provide an alternative betaine composition which can be produced in an efficient fashion and moreover can provide additional benefits.

The objective in achieved by means of a betaine composition, containing:
- between 40 and 85 wt.% betaine;
- between 1 and 20 wt.% fructan; and
- between 1 and 30 wt.% glucose and/or fructose.

Weight percentages as given herein are calculated on the basis of total dry matter, unless stated otherwise.

WO-A-2011/141175 discloses a process for the recovery of betaine from molasses. In example 1 of WO-A-2011/141175, a sugar beet molasses was treated with an enzyme having fructosyltransferase activity; subsequently, a chromatographic separation was done, leading a.o. to a fraction consisting essentially only of betaine.

WO 2013/072048 discloses a process for the recovery of betaine from a raw material consisting essentially of molasses, comprising: a demineralisation step, in which the overall amount of salts in the molasses is brought to a level lying below 2 wt.% (on overall dry matter); a conversion step, in which the molasses is subjected to the action of a fructan-forming enzyme, to form a fructan-containing molasses (fructan-molasses); a separation step, in which the fructan-molasses is subjected to a chromatographic separation, thereby obtaining a betaine-containing fraction.

Metzler-Zebeli, B. U. *et al.* (2009) describes the use of betaine and fructans in the nutrition of weaning pigs.

The invention relates to a betaine composition. As meant herein, betaine is used in its meaning of glycine betaine or N,N,N-trimethylglycine, a zwitterion found a.o. in sugar beets (*Beta vulgaris*) and having structural formula (I):

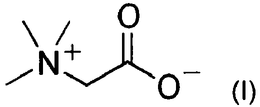

As is known, betaine has a number of functions in mammals, such as being a contributor to osmotic pressure and to function as methyl donor. These functions have led to the circumstance that there is a market for betaine, and it is thus desirable to obtain betaine as a product in an efficient way. One known group of sources of betaine is that of betaine-containing molasses, such as for example sugar beet molasses.

The betaine composition according to the invention contains at least 40 wt.% betaine. Preferably, betaine is the largest dry matter constituent of the betaine composition. More preferably, the betaine composition contains at least 45, 50, 55, 60, 65, or even at least 70 wt.% betaine.

In order to allow for the presence of further compounds so as to open the possibility for a betaine composition having additional benefits, the betaine composition according to the invention should contain at most 85 wt.% betaine. Preferably, the betaine composition according to the invention contains at most 83, 81, 79, 77, or even at most 75 wt.% betaine.

The betaine composition according to the invention should contain fructan. The term fructan as used herein has its common meaning of being a generic term that relates to a carbohydrate material consisting mainly of fructosyl-fructose links with optionally a glucose starting moiety. The meaning of fructan encompasses the more specific compounds inulin - wherein the fructosyl-fructose links are mainly of the β(2→1) type - and levan - wherein the fructosyl-fructose links are mainly of the β(2→6) type. Both inulins and levans can be linear or branched, and both can be in polydisperse form, i.e. in the form of a mixture of various degrees of polymerisation, or in homodisperse form.

Inulin is usually polydisperse, i.e. a mixture of compounds of various chain lengths whereby the degree of polymerisation (DP) of the individual compounds can range from 2 to 100 or higher. An individual inulin compound consisting of n fructose moieties is often represented with formula Fₙ, whereas an individual inulin compound having a glucose starting moiety and m fructose moieties is often represented with formula GFₘ. The term fructo-oligosaccharide - abbreviated as FOS - as used herein indicates a specific form of an inulin material, either monodisperse or polydisperse, whereby the DP of the individual compounds ranges from 2 to 10, in practice often from 2 to 9, or from 2 to 8 or from 2 to 7. Commercially available FOS is usually a polydisperse material having a number-averaged degree of polymerisation ( D̅P̅) of about 2 to 5. FOS compounds that were synthesised from sucrose typically consist for the majority of compounds having formula GFₘ, whereby m is the degree or polymerisation of the compound minus 1.

In practice, FOS is also referred to as oligofructose. As used herein, the terms fructo-oligosaccharide and oligofructose are considered to be synonyms.

The presence of fructan in the betaine composition of the invention can lead to certain benefits in use and may, when used in for example animal nutrition applications, contribute to one or more of effects known from prebiotics such as improved functioning of the gastro-intestinal system and/or the immune system. The fructan may also contribute to further types of benefits in use, such as a contribution to texture or viscosity; such benefits are as such known for fructans. The extent to which these benefits will show will depend on the specific way the betaine composition will be used.

The betaine composition according to the invention contains less fructan than betaine; in particular, it contains at least 1, 2, or at least 3 wt.% fructan. Preferably, the amount of fructan is higher than the amount of glucose and/or fructose in the betaine composition by at least 0.5 or even at least 1, 2, or 3 wt.%. More preferably, the amount of fructan is at least 5, 7, or even at least 9 wt.%.

The betaine composition according to the invention should contain at most 20 wt.% fructan. Preferably, the betaine composition according to the invention contains at most 18, 16, 14, or even at most 12 wt.% fructan.

The fructan in the betaine composition preferably consists essentially of inulin. More preferably, the fructan in the betaine composition consists of inulin. Most preferably, the fructan in the betaine composition consists essentially of FOS or even consists of FOS.

As used herein, the terms 'essentially', 'consist(ing) essentially of', 'essentially all' and equivalents have, unless noted otherwise, in relation to a composition or a process step the usual meaning that deviations in the composition or process step may occur, but only to such an extent that the essential characteristics and effects of the composition or process step are not materially affected by such deviations.

The betaine composition according to the invention should contain glucose and/or fructose. In one preferred embodiment, the betaine composition contains glucose and optionally fructose, preferably essentially no fructose or even no fructose. In an alternative preferred embodiment, the betaine composition contains fructose and optionally glucose, preferably essentially no glucose or even no glucose. The presence of glucose and/or fructose enriches the betaine composition with a source of energy. The total amount of glucose and/or fructose in the betaine composition should be at least 1.0 wt.%, preferably at least 1.5, 2.0, 2.5, 3.0, 3.5, or even at least 4.0 wt.%. In order to ensure that the benefits of betaine itself and the fructan can be present sufficiently, the total amount of glucose and/or fructose should be at most 30 wt.%, preferably at most 25, 20, 15, or 10 wt.%, more preferably at most 9.5, 9.0, 8.5, 8.0, 7.5, 7.0, 6.5, or even at most 6.0 wt%. In a preferred embodiment, the total amount of glucose and/or fructose is lower than the amount of fructan, preferably by at least 1 or even at least 2 wt.%.

In an embodiment of the invention, the amount of fructan in the betaine composition is at least 0.5 wt.% lower than the amount of betaine, and the total amount of glucose and/or fructose is at least 0.5 wt.% lower than the amount of fructan.

The betaine composition according to the invention may be prepared by combining the individual constituents in a desired ratio within the ranges as given herein, for example by means of mixing or blending. The betaine composition may be in the form of a powder mixture; the betaine composition according to the invention may also be in liquid form, for example in the form of an aqueous dispersion or solution.

In a preferred embodiment of the invention, the betaine composition according to the invention may be prepared from a sugar beet molasses, by first applying a conversion step as detailed in WO-A-141175, followed by a modified version of the chromatographic separation step as detailed in WO-A-141175. The modification of the separation step should be such that the betaine composition according to the present invention is obtained from the chromatographic separation. The goal of the modification having now been set herein, the modification itself may be achieved by the person skilled in the art via routine experiments.

In an embodiment of the betaine composition according to the invention, the betaine composition may contain some sucrose. Preferably, the amount of sucrose is up to 10 wt.%, preferably between 0.1 and 8 wt.%, more preferably between 0.5 and 6 wt.%. More preferably, the amount of sucrose is at most 5, 4, 3, or 2 wt.%. Most preferably, the amount of sucrose is lower than the total amount of glucose and/or fructose by at least 0.5 wt.%.

The invention furthermore relates to a glucose composition. Glucose compositions are as such known, and widely used in food, animal nutrition, and other applications. Known glucose compositions are often supplied in the form of a starch hydrolysate.

It is the objective of the present invention to provide an alternative glucose composition which can be produced in an efficient fashion and moreover can provide additional benefits.

The objective in achieved by means of a glucose composition, containing:
- between 40 and 72 wt.% glucose,
- between 0 and 25 wt.% fructose,
- between 0.5 and 30 wt.% fructan,
- between 0.05 and 5 wt.% betaine, and
- between 0 and 10 wt.% sucrose.

An advantage of the glucose composition of the invention is that it can bring several benefits beyond the delivery of the glucose as such, whereby such benefits are not found in known starch hydrolysates.

The glucose composition of the invention should contain at least 40 wt.% glucose. Preferably, the glucose composition contains at least 42, 44, 46, 48, 50, 52, 54, 56, 58, or even at least 60 wt.% glucose.

The glucose composition of the invention should contain at most 72 wt.% glucose. Preferably, the glucose composition contains at most 71, 70, 69, 68, 66, 64, or even at most 62 wt.% glucose.

The glucose composition of the invention may contain up to 25 wt.%, preferably up to 20, 15, or 10 wt.% fructose. In a preferred embodiment, the glucose composition of the invention contains at least 0.5, 1.0, 2.0, 2.5, or even at least 3.0 wt.% fructose. Preferably, the glucose composition of the invention contains at most 9.5, 9.0, 8.5, 8.0, 7.5, or even at most 7.0 wt.% fructose.

The glucose composition of the invention should contain at least 0.5 wt.%, preferably at least 1 wt.% fructan. Preferably, the glucose composition contains at least 1.5, 2.0, 2.5, 3.0, 3.5, or even at least 4.0 wt.% fructan.

The glucose composition of the invention should contain at most 30, preferably at most 25 wt.% fructan. Preferably, the glucose composition contains at most 24, 23, 22, 21, 20, 19, 18, 17, 16, 15, 14, 13, or even at most 12 wt.% fructan. In a preferred embodiment, the glucose composition contains more fructan than fructose by at least 0.5 wt.%.

The fructan in the glucose composition preferably consists essentially of inulin or even consists of inulin. More preferably, the fructan in the glucose composition consists essentially of FOS or even consists of FOS.

The glucose composition of the invention should contain at least 0.05 wt.% betaine. Preferably, the glucose composition contains at least 0.10, 0.15, or even at least 0.20 wt.% betaine.

The glucose composition of the invention should contain at most 5 wt.%, preferably at most 4, 3, or 2 wt.% betaine. More preferably, the glucose composition contains at most 1.8, 1.7, 1.6, 1.5, 1.4, 1.3, 1.2, 1.1, or even at most 1.0 wt.% betaine. In a preferred embodiment, the glucose composition contains less betaine than fructan by at least 0.5 wt.%. If the glucose composition according to the invention contains fructose, it is preferred that the amount of betaine is lower than the amount of fructose, preferably by at least 0.1 wt.%.

The glucose composition of the invention may contain up to 10 wt.% sucrose. Preferably, the glucose composition contains at most 9.0, 8.0, 7.0, 6.5, 6.0, 5.5, 5.0, 4.5, or even at most 4.0 wt.% sucrose.

Preferably, the glucose composition of the invention contains at least 0.5, 1.0, 1.5, 2.0, or even 2.5 wt.% sucrose.

The glucose composition of the invention should preferably contain no disaccharides, oligosaccharides, or polysaccharides obtainable by the hydrolysis of starch, or at most 5, 4, 3, 2, 1, 0.5, or even 0.1 wt.% thereof. Examples of such compounds are maltose and maltotriose. The hydrolysis of starch is as such well-known, and can be executed by means of for example acidic hydrolysis or enzymatic hydrolysis.

It was found that the combination of compounds, and the benefits associated with them, in the glucose composition of the invention creates a high suitability for use in calf milk replacer compositions. The invention therefore also relates to a calf milk replacer composition, containing between 1 and 40 wt.% of the glucose composition of the invention. In a preferred embodiment of the invention, the glucose composition contains essentially no iron or even contains no iron.

The glucose composition of the invention may be prepared by combining the individual constituents in a desired ratio within the ranges as given herein. The glucose composition may be in the form of a powder mixture; the glucose composition according to the invention may also be in liquid form, for example in the form of an aqueous dispersion or solution.

In a preferred embodiment of the invention, the glucose composition according to the invention may be prepared from a sugar beet molasses.

The term molasses as used herein has its common meaning of being a by-product formed in a process for the preparation of sucrose, in particular in the crystallisation stages; furthermore, the molasses as used in the process according to the invention should contain betaine. As used herein, the term molasses refers to the molasses as obtained in the process for the preparation of sucrose, or to a concentrated or a diluted form thereof, whereby the dilution is preferably done with an aqueous phase. The term molasses as meant herein also encompasses a molasses that has been subjected to one or more pre-treatments while still continuing to contain significant amounts of sucrose, betaine, and salts. An example of such a pre-treatment is the reduction of the amount of sucrose by between 10, 20, or 30 and 50, 60, or even 70%; another example of such a pre-treatment is a so-called softening step, aimed at reducing the amount of calcium by at between 10, 20, 30, or 40 and 50, 60, or even 70%. In one embodiment, the molasses is diluted with water; in another embodiment, the molasses is diluted with a vinasse. In one embodiment, the molasses is wholly replaced with a partially fermented vinasse, whereby the vinasse should have a sufficient amount of sucrose in order for the conversion step to take place. Preferably, the molasses is sugar beet molasses. As is known, sugar beet molasses can typically contain, based on total weight of the undiluted and non-pre-treated form: between 45 and 65 wt.% of sucrose; typically between 3 and 8 wt.% of betaine; typically between 6 and 10 wt.% of amino acids, peptides, or proteins; smaller amounts of about 1 wt.% of non-sucrose carbohydrates like fructose and glucose; and a significant amount of other compounds such as organic salts and inorganic salts.

The glucose composition of the invention may preferably be prepared from a molasses, by first applying a conversion step as detailed in WO-A-141175, followed by a modified version of the chromatographic separation step as detailed in WO-A-141175. The modification of the separation step should be such that the glucose composition according to the present invention is obtained from the chromatographic separation. The goal of the modification having now been set herein, the modification itself may be achieved by the person skilled in the art via routine experiments.

In an embodiment of the invention, the betaine composition of the invention and the glucose composition of the invention are prepared simultaneously from a sugar beet molasses in a process comprising:
- a conversion step, in which the molasses is subjected to the action of a fructan-forming enzyme, to form a fructan-containing molasses; and
- a recovery step, in which the fructan-containing molasses is subjected to a chromatographic separation, such that a betaine composition, a glucose composition, and a fructan composition are recovered.

A conversion step as meant herein is as such known, from for example the conversion step as disclosed in WO-A-2011/141175. In the conversion step according to the invention, the molasses is subjected to the action of a fructan-forming enzyme. This may be achieved by means as such known. The molasses may be present as such or in concentrated or diluted form; preferably, the molasses is present in diluted form, the dilution preferably having been done with water. If a certain dilution, or an increase of dilution, leads to a reduction of the efficiency of the enzyme used, then the benefit of dilution should be balanced against the efficiency reduction by the skilled person in routine fashion in order to establish the optimum for the specific circumstances. In one embodiment, the appropriate enzyme is in free form and is thoroughly mixed with the molasses; the enzyme-containing molasses is brought to conditions of temperature and pH such that the enzyme shows appreciable activity. Alternatively, the molasses is first brought to conditions of temperature and pH such that the enzyme can show appreciable activity, followed by the admixture of the enzyme. In another embodiment, the enzyme is available in immobilized form, and the molasses is made to flow along the immobilized enzyme while also having been brought to appropriate conditions of temperature and pH.

The enzyme used in the process according to the invention should be able to catalyse the formation of fructans from sucrose. Free glucose may be formed as by-product.

The formation of fructan from sucrose may be achieved by selecting an enzyme having fructosyltransferase activity. Such enzymes are as such known, for instance as categorised under enzyme category number EC 2.4.1.99 or EC 2.4.1.9. An early disclosure of such an enzyme is in "The Production of Fructooligosaccharides from Inulin or Sucrose Using Inulinase or Fructosyltransferase from Aspergillus ficuum", Barrie E. Norman & Birgitte Højer-Pedersen, Denpun Kagaku vol 36, No. 2, pp 103-111 (1989).

Furthermore, it is known that some β-fructofuranosidases or invertases, i.e. enzymes categorised under EC 3.2.1.26, can also have fructosyltransferase activity and thus could be suitable in the process according to the invention.

Moreover, also enzymes having an endo-inulinase activity - such as enzymes classified under EC 3.2.1.7 - may in the presence of sucrose give rise to the formation of fructans such as FOS, in particular if they act in a mixture having a high sucrose content of 40 or 50 wt.% sucrose or higher.

Yet furthermore, enzymes having levansucrase activity - such as enzymes classified under EC 2.4.1.10 - can be suitable for use in the method according to the invention.

Also, enzymes having inulin synthase activity, such as for example the enzymes disclosed in EP-A-1298204, can be suitable for use in the method according to the invention.

One example of a preferred enzyme for use in the conversion step of the invention is the endo-inulinase Novozyme 960 (supplier: Novozymes). Another example of a preferred enzyme for use in the conversion step of the invention is Pectinex Ultra SP-L (supplier: Novozymes). It is according to the invention also possible that the enzyme constitutes a combination of two or more enzymes having fructosyltransferase and/or endo-inulinase activity.

In an embodiment of the invention, the molasses is brought in contact with an enzyme capable of catalyzing the formation of fructo-oligosaccharide (FOS) from sucrose. This embodiment thus relates to a process according to the invention wherein in the conversion step, the molasses is subjected to the action of an enzyme having endo-inulinase activity and/or fructosyltransferase activity to form a fructo-oligosaccharide-containing molasses (FOS-molasses), and wherein the recovery step is executed on the FOS-molasses.

The amount of enzyme needed in the process according to the invention depends on various - as such known - factors such as process temperature, amount of raw materials, pH, allowable process duration, and desired conversion rates. These and other relevant factors may be determined for the process of the invention by the person skilled in the art following the generally accepted procedures in this technical field.

In the process according to the invention, the enzyme is allowed to act on the molasses for a period of time that is sufficiently long to create a fructan-containing molasses, preferably a FOS-containing molasses. The duration of execution of this step according to the invention is mainly chosen in function of the amount of fructan, preferably FOS that is desired. As the skilled person knows, this duration is often in the range between 0.5 or 1 and 72 hours, preferably between 1.5 or 2 and 50 hours, more preferably between 3 or 4 and 36 hours, during which a fructan-containing molasses, preferably a FOS-containing molasses is formed.

It is preferred that in the conversion step between 5 wt.% and 100 wt.% of the sucrose in the molasses is converted. More preferably, at least 10, 20, 30, 40, 50, 60, 70, 80, or 90 wt.% of the sucrose is converted. It is particularly preferred to convert essentially all sucrose. It was found that if the percentage of sucrose that is converted is increased, the subsequent recovery of betaine can be executed more efficiently.

Upon completion of the formation of the fructan-containing molasses, preferably the FOS-containing molasses, and in case a free, non-immobilized enzyme was used and mixed into the molasses, it may be desirable to ensure that the enzyme is deactivated. If this is the case, then an enzyme deactivating step may be implemented. The deactivation of the enzyme may be achieved by methods that are as such known and may differ for each specific type of enzyme. An example of such a method of deactivation is an increase in temperature - to a level of for example about 80, 85 or 90°C - followed by a residence time of between 5 and 30 minutes at such an increased temperature. A further benefit of exposure at such a temperature is that the amounts of any bacteria that may be present are reduced. A further example of a method of enzyme deactivation is a UHT (ultra-pasteurisation) treatment.

The recovery step of the invention is executed either during the conversion step or subsequent to the conversion step. Preferably, the recovery step is executed subsequent to the conversion step. In the recovery step, the fructan-molasses is subjected to a chromatographic separation. As is known, the subjection of a material to a chromatographic separation can lead to the splitting of the material into various fractions. The recovery step according to the invention should be done such that at least three fractions are formed, namely a betaine composition according to the invention, a glucose composition according to the invention, and a fructan composition. It is known to the person skilled in the art that the particular choice of the stationary phase in the chromatographic separation can influence the performance of the recovery step. The chromatographic separation may be executed by means that are as such known, such as the passing of the fructan-molasses over a resin.

In a main embodiment of the invention, the recovery step is done via the use of resins that are typical for ion-exchange chromatography. As is known, a variety of resins is available for this purpose. In one preferred embodiment of the process of the invention, a strong acid cation exchange resin is chosen. One example of such resins are Styrene-DVB resins, i.e. resins having structures based on styrene copolymerised with divinylbenzene DVB, such as Dowex™ resins.

In the recovery step the purpose of using ion exchange resins is not primarily to exchange ions - in fact, the fructan-molasses will preferably have a very low amount of ions. Rather, it was found that ion exchange resins can favourably influence the separation efficiency towards obtaining a betaine composition, a glucose composition, and other useful fractions such as a fructan composition. It was also found that if a (strong acid) cation exchange resin is chosen, the choice of cation can influence the separation efficiency. In one embodiment of the invention, cation exchange resins essentially in the sodium form are preferred. In a further embodiment of the invention, cation exchange resins essentially in the potassium form are preferred. In yet a further embodiment of the invention, cation exchange resins essentially in the calcium form are preferred. Further preferred embodiments include resins in magnesium or iron form.

In a main embodiment of the invention, cation exchange resins in mixed form are used; this means that the recovery step is done by using a resin system consisting of a mixture of resins in different form, i.e. differing at least in the cation form they are in. Preferred choices of cations in this main embodiment are magnesium, iron, sodium, potassium, and calcium. More preferably, sodium, potassium, and calcium are used.

It was found that an increase of the presence of resins in the calcium form can contribute to an improved separation efficiency of betaine; it was also found, however, that a very high percentage of resin in the calcium form can lead to a situation that the release of betaine from the resins is tailing very far behind the release of all other compounds in the molasses; while this may be a positive feature in case it is desirable to recover a high-purity betaine-containing fraction, it should according to the invention be balanced such that a betaine composition according to the invention is recovered. In one preferred embodiment, therefore, a cation exchange resin system is used wherein between 5 and 80 wt.% of the resin system consists of resins in the calcium form; it is then preferred that between 95 and 20 wt.% of the resin system consists of resins in the sodium and/or potassium form. Preferably, between 6, 7, or 8 and 78, 76, 74, 72, or 70, or between 10 and 65 or 60, or between 12 or 14 and 55 or 50, or between 15 and 45 or 40, or between 18, 20 or 22 and 35 or 30 wt.% of the resins in the resin system are in the calcium form. Correspondingly, it is then preferred that between 22, 24, 26, 28, or 30 and 94, 93, or 92, between 35 and 40 and 90, between 45 or 50 and 88 or 86, between 55 or 60 and 85, or between 65 or 70 and 82, 80, or 78 wt.% of the resins in the resin system are then in the sodium and/or potassium form. The choice of mixture of the cation form of the resin should be determined via routine optimisation, with the goal of achieving the betaine composition and glucose composition according to the invention. The formation of the betaine composition according to the invention and the glucose composition according to the invention will typically be accompanied with the formation of a rest-composition having a significant amount of fructan, as follows from the mass balance of the process. The rest-composition is herein named fructan composition.

As is known in case a resin is used in the recovery step, a certain routine optimization may be needed in order to choose the optimal type of resin, e.g. by varying the degree of cross-linking in the resin.

Preferably, the chromatographic separation is done in a simulated moving bed (SMB) system, or further developments of SMB systems such as a Sequential Simulated Moving Bed (SSMB) or an Improved Simulated Moving Bed (ISMB). This has the advantage that the recovery step may be done on a continuous basis. In one embodiment, a system is chosen that allows the simultaneous production of multiple fractions, such as the NMCI system.

In view of the preference of having and maintaining a certain desired cation composition in the recovery step of the invention, it may be desirable to execute a demineralisation step prior to executing the recovery step. In the demineralisation step the overall amount of salts, both organic and inorganic, in the molasses is brought to a level lying below 2 wt.%.

Demineralisation steps are known as such; one way of executing this step is by means of chromatography, using for example resins such as ion-exchange resins. The demineralisation step may be executed in the form of two subsequent steps, a first 'main' step and a second 'polishing' step. As is known to the person skilled in the art, ion-exchange resins such as strong acid cation exchange resins may be used in demineralisation steps; it is noted hereby that typically the cations in the resin are not primarily present with the purpose of being exchanged, but rather to serve in aiding to achieve a separation of ionic compounds such as salts from non-ionic compounds such as carbohydrates. It is preferred that if cationic resins are used in the demineralisation step, the cation form of the resin is primarily a reflection of the main cation or cations as present in the molasses; often in practice, this will mean that resins in potassium and/or sodium form can favourably be used. It was found that in the demineralisation step, betaine typically goes into the fraction of the non-ionic compounds, even though it is a zwitter ion.

The demineralisation step may also be executed by means of a chromatography comprising a combination of cation and anion exchangers in series; this embodiment is often also suitable as 'polishing' step.

Preferably, the overall amount of salts in the molasses is brought to a level lying below 2.0, 1.5, 1.0, 0.75, 0.60, 0.50, 0.40, 0.30, 0.25, 0.20, 0.15, 0.10, or even below 0.08, 0.05, 0.04, 0.03, 0.02, or 0.01 wt.% of overall dry matter in the molasses. At the same time, any loss of betaine from the molasses into the fraction or fractions containing the salts that are separated off from the molasses should be kept to a minimum. Preferably, at most 40, 35, 30, 25, 20, or even at most 15 or 10 wt.% of the total amount of betaine that entered the demineralisation step is lost into the fraction or fractions containing the salts that are separated off from the molasses. Similarly, the loss of carbohydrates from the molasses into the fraction or fractions containing the salts that are separated off from the molasses should be kept to a minimum. Preferably, at most 40, 35, 30, 25, 20, or even at most 15 or 10 wt.% of the total amount of carbohydrates that entered the demineralisation step are lost into the fraction or fractions containing the salts that are separated off from the molasses.

The limiting of losses of betaine and/or carbohydrates from the molasses in the demineralisation step may be achieved via means that are as such known, such as preferably via the routine optimisation of a chromatographic separation.

It was found that a reduction of the amount of salts in the molasses can have a beneficial effect on the efficiency of the recovery step, executed later in the process of the invention. Furthermore, it was found that the demineralisation step can have the advantage that food-quality products, i.e. products suitable for human consumption, can be prepared more efficiently. Thus, the demineralisation step should preferably be executed in such a way, that cations in the molasses are removed to the extent that any remaining cations will not significantly affect the cation composition of the resin system in the recovery step. Preferably, the demineralisation step is executed such that the fractions obtained in the recovery step have, when put into water at 28 wt.%, a conductivity at most 2 mS/cm, preferably at most 1.5, 1, or 0.5 mS/cm. More preferably, the conductivity is even lower with a value of at most 400, 300, 200, or even 100 µS/cm.

The demineralisation step may in an alternative embodiment be executed partly or fully by means of electrodialysis. Electrodialysis is as such known; WO-A- 2007/071727, for example, discloses that a sugar beet molasses is treated in an electrodialysis step with the purpose of removing ions from the molasses. It was found that electrodialysis can be successfully applied to a fructan-containing molasses.

The demineralisation step may in a further embodiment be executed as a combination of electrodialysis and chromatography.

If a demineralisation step is executed, it should be done prior to the execution of the recovery step. In one embodiment, it is executed subsequent to the conversion step. In an alternative embodiment, the demineralisation step is executed prior to the conversion step; it was found that the loss of sucrose into the salt-containing fraction or fractions is often lower than the loss of fructans into the salt-containing fraction or fractions.

## Claims

1. Betaine composition, containing:
• between 40 and 85 wt.% betaine;
• between 1 and 20 wt.% fructan; and
• between 1 and 30 wt.% glucose and/or fructose.

2. Betaine composition according to claim 1, further containing between 0 and 10 wt.% sucrose.

3. Betaine composition according to claim 1 or 2, wheren the amount of fructan is at least 0.5 wt.% lower than the amount of betaine, and wherein the total amount of glucose and/or fructose is at least 0.5 wt.% lower than the amount of fructan.

4. Betaine composition according to any one of claims 1 - 3, wherein the fructan consists essentially of fructo-oligosaccharides.

5. Glucose composition, containing:
• between 40 and 72 wt.% glucose;
• between 0 and 25 wt.% fructose;
• between 0.5 and 30 wt.% fructan;
• between 0.05 and 5 wt.% betaine; and
• between 0 and 10 wt.% sucrose.

6. Glucose composition according to claim 5, wherein the fructan consists essentially of fructo-oligosaccharides.

7. Glucose composition according to claim 5 or 6, containing at most 5 wt.% disaccharides, oligosaccharides, or polysaccharides obtainable by the hydrolysis of starch.

8. Calf milk replacer composition, containing between 1 and 40 wt.% of the glucose composition of any one of claims 5 - 7.

9. Process for the preparation of a betaine composition according to any one of claims 1 - 4 and a glucose composition according to any one of claims 5 - 7 from a sugar beet molasses, comprising:
• a conversion step, in which the molasses is subjected to the action of a fructan-forming enzyme, to form a fructan-containing molasses; and
• a recovery step, in which the fructan-containing molasses is subjected to a chromatographic separation, such that a betaine composition, a glucose composition, and a fructan composition are recovered.

10. Process according to claim 9, further comprising a demineralisation step in which the overall amount of salts, both organic and inorganic, in the molasses or the fructan-containing molasses is brought to a level lying below 2 wt.%, whereby the demineralisation step is executed prior to the recovery step.

11. Process according to claim 10, wherein the demineralisation step is executed prior to the conversion step.

## Patentansprüche

1. Betainzusammensetzung, enthaltend:
• 40 bis 85 Gew.-% Betain;
• 1 bis 20 Gew.-% Fructan; und
• 1 bis 30 Gew.-% Glucose und/oder Fructose.

2. Betainzusammensetzung nach Anspruch 1, ferner 0 bis 10 Gew.-% Fructose enthaltend.

3. Betainzusammensetzung nach Anspruch 1 oder 2, wobei die Menge an Fructan mindestens 0,5 Gew.-% geringer als die Menge an Betain ist und wobei die Gesamtmenge an Glucose und/oder Fructose mindestens 0,5 Gew.-% geringer als die Menge an Fructan ist.

4. Betainzusammensetzung nach irgendeinem der Ansprüche 1 - 3, wobei das Fructan im Wesentlichen aus Fructooligosachariden besteht.

5. Glucosezusammensetzung, enthaltend:
• 40 bis 72 Gew.-% Glucose;
• 0 bis 25 Gew.-% Fructose;
• 0,5 bis 30 Gew.-% Fructan;
• 0,05 bis 5 Gew.-% Betain; und
• 0 bis 10 Gew.-% Sucrose.

6. Glucosezusammensetzung nach Anspruch 5, wobei das Fructan im Wesentlichen Fructooligosachariden besteht.

7. Glucosezusammensetzung nach Anspruch 5 oder 6, die höchstens 5 Gew.-% Disaccharide, Oligosaccharide oder Polysaccharide, die durch Hydrolyse von Stärke erhältlich sind, enthält.

8. Kalbsmilchersatzzusammensetzung, die 1 bis 40 Gew.-% der Glucosezusammensetzung nach irgendeinem der Ansprüche 5 - 7 enthält.

9. Verfahren für die Herstellung einer Betainzusammensetzung nach irgendeinem der Ansprüche 1 - 4 und einer Glucosezusammensetzung nach irgendeinem der Ansprüche 5 - 7 aus einer Zuckerrübenmelasse, umfassend:
• einen Umwandlungsschritt, wobei die Melasse der Wirkung eines fructanbildenden Enzyms unterworfen wird, um eine fructanhaltige Melasse zu bilden; und
• einen Gewinnungsschritt, wobei die fructanhaltige Melasse einer chromatographischen Trennung unterworfen wird, derart, dass eine Betainzusammensetzung, eine Glucosezusammensetzung und eine Fructanzusammensetzung gewonnen werden.

10. Verfahren nach Anspruch 9, ferner einen Entmineralisierungsschritt umfassend, wobei die Gesamtmenge an Salzen, sowohl organischen als auch anorganischen, in der Melasse oder der fructanhaltigen Melasse auf ein Niveau gebracht wird, das unter 2 Gew.-% liegt, wobei der Entmineralisierungsschritt vor dem Gewinnungsschritt ausgeführt wird.

11. Verfahren nach Anspruch 10, wobei der Entmineralisierungsschritt vor dem Umwandlungsschritt ausgeführt wird.

## Revendications

1. Composition à base de bétaïne, contenant :
• entre 40 et 85 % en poids de bétaïne ;
• entre 1 et 20 % en poids de fructane ; et
• entre 1 et 30 % en poids de glucose et/ou de fructose.

2. Composition à base de bétaïne selon la revendication 1, contenant en outre entre 0 et 10 % en poids de saccharose.

3. Composition à base de bétaïne selon la revendication 1 ou 2, dans laquelle la quantité de fructane est au moins 0,5 % en poids inférieure à la quantité de bétaïne et dans laquelle la quantité totale de glucose et/ou de fructose est au moins 0,5 % en poids inférieure à la quantité de fructane.

4. Composition à base de bétaïne selon l'une quelconque des revendications 1 à 3, dans laquelle le fructane est constitué essentiellement de fructo-oligosaccharides.

5. Composition à base de glucose, contenant :
• entre 40 et 72 % en poids de glucose ;
• entre 0 et 25 % en poids de fructose ;
• entre 0,5 et 30 % en poids de fructane ;
• entre 0,05 et 5 % en poids de bétaïne ; et
• entre 0 et 10 % en poids de saccharose.

6. Composition à base de glucose selon la revendication 5, dans laquelle le fructane est constitué essentiellement de fructo-oligosaccharides.

7. Composition à base de glucose selon la revendication 5 ou 6, contenant au maximum 5 % en poids de disaccharides, d'oligosaccharides ou de polysaccharides pouvant être obtenus par l'hydrolyse d'amidon.

8. Composition de produit de remplacement de lait pour les veaux, contenant entre 1 et 40 % en poids de la composition à base de glucose selon l'une quelconque des revendications 5 à 7.

9. Procédé pour la préparation d'une composition à base de bétaïne selon l'une quelconque des revendications 1 à 4 et d'une composition à base de glucose selon l'une quelconque des revendications 5-7 à partir d'une mélasse de betterave à sucre, comprenant :
• une étape de conversion, dans laquelle la mélasse est soumise à l'action d'une enzyme formant du fructane, pour former une mélasse contenant du fructane ; et
• une étape de récupération, dans laquelle la mélasse contenant du fructane est soumise à une séparation chromatographique, de telle façon qu'une composition à base de bétaïne, une composition à base de glucose et une composition à base de fructane sont récupérées.

10. Procédé selon la revendication 9, comprenant en outre une étape de déminéralisation dans laquelle la quantité globale de sels, aussi bien organiques qu'inorganiques, dans la mélasse ou la mélasse contenant du fructane est amenée à un niveau se situant au-dessous de 2 % en poids, l'étape de déminéralisation étant exécutée avant l'étape de récupération.

11. Procédé selon la revendication 10, dans lequel l'étape de déminéralisation est exécutée avant l'étape de conversion.
